(19) Europäisches Patentamt
European Patent Office
Office européen des brevets

(11) EP 2 001 494 B1

(12) EUROPÄISCHE PATENTSCHRIFT

(45) Veröffentlichungstag und Bekanntmachung des Hinweises auf die Patenterteilung:
20.01.2010 Patentblatt 2010/03

(51) Int Cl.:
*A61K 36/708* (2006.01)     *B01D 11/02* (2006.01)

(21) Anmeldenummer: 07722138.0

(22) Anmeldetag: 30.03.2007

(86) Internationale Anmeldenummer:
PCT/DE2007/000580

(87) Internationale Veröffentlichungsnummer:
WO 2007/112731 (11.10.2007 Gazette 2007/41)

(54) **WIRKSTOFFFRAKTIONEN MIT HOHER ANTIVIRALER AKTIVITÄT**

ACTIVE SUBSTANCE FRACTIONS OF HIGH ANTIVIRAL ACTIVITY

FRACTIONS DE PRINCIPE ACTIF A PUISSANTE ACTIVITE ANTIVIRALE

(84) Benannte Vertragsstaaten:
AT BE BG CH CY CZ DE DK EE ES FI FR GB GR HU IE IS IT LI LT LU LV MC MT NL PL PT RO SE SI SK TR

(30) Priorität: 03.04.2006 DE 102006015573

(43) Veröffentlichungstag der Anmeldung:
17.12.2008 Patentblatt 2008/51

(73) Patentinhaber:
• Hochschule Anhalt (FH)
06366 Köthen (DE)
• ESA Patentverwertungsagentur Sachsen-Anhalt GmbH
39114 Magdeburg (DE)

(72) Erfinder:
• KABRODT, Kathrin
06406 Bernburg (DE)

• SCHELLENBERG, Ingo
06847 Dessau (DE)

(74) Vertreter: Fischer, Volker
Fritz-Reuter-Strasse 7
39108 Magdeburg (DE)

(56) Entgegenhaltungen:
EP-A- 0 568 001     DE-A1- 10 132 936
US-A- 4 670 265

• DATABASE BIOSIS [Online] BIOSCIENCES INFORMATION SERVICE, PHILADELPHIA, PA, US; 1996, WANG ZHIYU WANG GUITING ET AL: "Anti-herpes virus action of ethanol-extract from the root and rhizome of Rheum officinale Baill" XP002443326 Database accession no. PREV199699156976 in der Anmeldung erwähnt & ZHONGGUO ZHONGYAO ZAZHI, Bd. 21, Nr. 6, 1996, Seiten 364-366, 384, ISSN: 1001-5302

**EP 2 001 494 B1**

## Beschreibung

Die Erfindung betrifft Wirkstofffraktionen mit hoher antiviraler Aktivität zur Virusinaktivierung und Desinfektion.

Die antivirale Wirkung kondensierter Gerbstoffe wurde bereits beschrieben und auch therapeutisch genutzt. Esquenazi, D., Wigg, M. D. u.a. (2002), Research in Microbiology 153: 647-652 untersuchten die antivirale Wirkung kondensierter Gerbstoffe, die aus den getrockneten Fasern der reifen Kokosnuss (Cocos nucifera L.) extrahiert wurden, an infizierten HEp-2 (Kehlkopfkrebszelllinie) und VERO-Zellkulturen. Sie konnten nachweisen, dass aus Catechinen aufgebaute Polyphenole die Aktivität eines Acyclovir resistenten Herpes simplex Virus Typ 1 (HSV-1-ACVr) hemmen. Es wurden sowohl ein Gesamtextrakt als auch mehrere chromatografisch vorgetrennte Fraktionen eingesetzt. Der Gesamtextrakt und eine Fraktion mit monomeren bis trimeren Procyanidinen zeigten die stärkste antivirale Aktivität, die von den Autoren mit einer Störung der Adsorption der Viren an den Zelllinien begründet wird.

Cheng, H. Y., Lin, C. C. u.a. (2002), Antivir Chem Chemother. 13(4): 223-229 untersuchten die antiviralen Eigenschaften von Prodelphinidin B-2 -3'-O-gallat, isoliert aus grünem Tee, auf Herpes Simplex Typ 2 kultiviert in VERO-Zellen. Die signifikante Reduktion der Infektion wird neben der durch den Wirkstoff verursachten Störung der Adsorption an die Wirtszelle auch auf eine Beeinträchtigung der Penetration des Virus zurückgeführt. Die gleichen Resultate wurden auch für die digalloylierte Verbindung Prodelphinidin B-2 -3,3'-O-gallat, isoliert aus Myrica rubra, am gleichen Virusmodell durch Cheng, H. Y., Un, T.C. u.a. (2003) Planta Med. 69(10): 953-956 erzielt. Antivirale Aktivität gegen einen Adenovirus zeigten verschiedene aus grünem Tee isolierte monomere Flavan-3-ole, insbesondere EGCG, aufgezeigt durch Weber u. a., (2003), Antiviral Res. 58(2):167-173). Die höchste Effektivität wurde erzielt, wenn EGCG appliziert wurde beim Übergang von der frühen zur späten Phase der viralen Infektion.

Die antivirale Aktivität bei aus Crataegus sinaica isolierten Flavonoiden und Proanthocyanidinen war besonders ausgeprägt im Bereich der dimeren bis tetrameren PC. Hier konnte eine vollständige HSV-1 Titerreduktion beobachtet werden. Die antivirale Aktivität wurde über die Inhibierung cytopathischer Effekte auf mit Herpes Simplex Virus infizierten VERO-Zellen bestimmt, beschrieben durch Shahat, A. A. u.a. (2002), Planta Med 68: 539-541 .

Die antivirale Aktivität gegen einen Influenza-Virus von mit Polyphenolen (Tanninen, Flavonoiden, Catechinen und Proanthocyanidinen) angereicherten Extrakten aus Geranium sanguineum L. wurde von Sokmen, M.u. a. (2005), Ufe Sciences 76: 2981-2993 untersucht. Dabei wurden ein methanolischer Gesamtextrakt sowie daraus gewonnene Ethylacetat- und Butanol-Fraktionen eingesetzt, um die Reproduktion von humanen Influenza-Viren (A/Aichi Virus in MDCK= Madin-Darby canine kidney-Zellen) in vitro zu beeinflussen. Gemessen wurde die Reduktion der cytopathischen Effekte. Sowohl der Gesamtextrakt als auch die BuOH-Fraktion zeigten eine signifikante Reduzierung der virusinduzierten cytopathischen Effekte.

Ein ethanolischer Extrakt aus den unterirdischen Teilen von Rh. officinale zeigte in vitro antivirale Aktivität gegen HSV. Die minimal inhibierende Dosis lag bei 100 $\mu$g/ml, beschrieben durch Wang, Z. W. u.a. (1996), Zhongguo Zhong Yao Za Zhi 21 (6): 364-366.

Obwohl es eine Reihe von gut charakterisierten antiviralen Wirkstoffen und Formulierungen gibt, besteht weiterhin die Anforderung nach neuen Wirksubstanzen. Zum einen weisen die bekannten Wirkstoffe eine ungenügende Aktivität auf oder werden nicht gut vertragen, zum anderen werden immer mehr Virusstämme resistent.

Die bekannten Extraktformulierungen aus Rheum-species- Arten zeigen unzureichende antivirale Wirkung.

Das Ziel der Erfindung besteht in der Bereitstellung von Wirkstofffraktionen aus Rheum-species- Arten mit gesteigertem Wirkstoffpotenzial zur Herstellung antiviraler Formulierungen.

Der Erfindung liegt die Aufgabe zu Grunde, Wirkstofffraktionen mit hoher antiviraler Aktivität bereitzustellen, die sich durch gute Verträglichkeit auszeichnen. Erfindungsgemäß wird diese Aufgabe durch Wirkstofffraktionen mit hoher antiviraler Aktivität aus Rheum-species-Arten gelöst, die dadurch gekennzeichnet sind, dass die Wirkstofffraktionen durch Extraktion der getrockneten und gemahlenen Wurzelproben mit Methanol erhalten, unter vollständiger Methanolentfernung die gebildete wässrige Phase von lipophilen Begleitstoffen befreit, die gereinigte wässrige Phase mit Ethylacetat ausgeschüttelt und die vereinigte Ethylacetatphase in eine begrenzte Anzahl von Wirkstofffraktionen säulenchromatografisch aufgetrennt und die Wirkstofffraktionen mit gleichem Inhaltsspektrum zusammengefasst werden.

Als außerordentlich überraschend hat sich herausgestellt, dass aus dem Ethylacetat Gesamtextrakt von Rheum-spec. durch chromatografische Auftrennung gewonnene einzelne Rhabarberfraktionen eine signifikante Virusinaktivierung bewirken.

Als Viren bezeichnet man in der Biologie genetische Elemente in Form von Nukleinsäuren, die als Fremdbestandteile in Zellen von Lebewesen (Wirtszellen) unabhängig von deren eigenen Nukleinsäuren mit Hilfe der Replikationseinrichtungen dieser Zellen repliziert werden. Virus-Nukleinsäuren sind entweder Desoxyribonukleinsäuren (DNA) oder Ribonukleinsäuren (RNA). Viren können als Nukleinsäure in der Wirtszelle und als freies Partikel außerhalb von Zellen vorkommen, wobei letztere Form als Virion bezeichnet wird.

Virionen bestehen aus einem Nukleinsäuremolekül, das von einer Hülle (Capsid) umgeben ist. Bei einigen Viren besitzen die Virionen außer der Proteinhülle noch weitere Bestandteile, z.B. eine Lipoproteinhülle.

Nachfolgend werden die für die Untersuchung der antiviralen Aktivität von polyphenolischen Fraktionen aus Rhabarber verwendeten Viren oder entsprechende Modellviren vorgestellt:

Parvoviren

Das porcine Parvovirus (PPV) wurde als Modellvirus für das humanpathogene Parvovirus B19 verwendet. Das porcine Parvovirus zählt zur Familie der Parvoviridae und weist eine Größe von 18-25 nm auf. Die Viruscapside haben einen ikosaedrischen Aufbau.

Die Viruspartikel bestehen aus 60 Capsomeren und haben an den Ecken etwa 7 nm lange Proteinvorsprünge. Die Virionen sind nicht von einer Membran umhüllt. Im Inneren der Capside ist das einzelsträngige, lineare, virale DNA-Genom enthalten.

Flaviviren

Das Hepatitis-C-Virus wird der Gruppe der Flaviviren zugeordnet. Da HCV nur unter enormen Aufwand in Zellkulturen vermehrt werden kann, ist man in Virusinaktivierungsstudien auf den Einsatz von Modellviren angewiesen. Die Genomstruktur des HCV weist auf eine Zugehörigkeit zur Familie der Flaviviren hin. Dabei kann es, wie das Virus der bovinen Diarrhoe (BVDV), dem Genus Pestivirus zugeordnet werden. Die infektiösen Viren der bovinen Diarrhoe haben einen Durchmesser von 40-50 nm. Die sphärischen Capside sind von einer Hüllmembran umgeben. Das Genom besteht aus einzelsträngiger RNA.

Togaviren

Ebenfalls als Modellvirus für HCV wurden das Semliki-Forest-Virus (SFV), welches zur Familie der Togaviren zählt, eingesetzt. Die infektiösen Partikel haben einen Durchmesser von 60-80 nm und bestehen aus ikosaedrischen oder sphärischen Capsiden, die von einer Membranhülle umgeben sind. Das Genom der Togaviren besteht aus einzelsträngiger RNA.

Herpesviren

Durch humanpathogene Herpesviren können latente Infektionen hervorgerufen werden. Nach Infektion können Herpesviren lebenslang in Leukozyten persistieren. Das Pseudorabiesvirus (PRV) wurde für diese Untersuchungen als Modellvirus für Herpesviren verwendet.

Die Virionen der Herpesviren haben einen Durchmesser von 150-200 nm und bestehen aus insgesamt mehr als 30 Strukturproteinen. Das Genom liegt in den Virionen als lineare, doppelsträngige DNAvor.

Die antivirale Wirkung von Gerbstoffen ist auf deren Interaktion mit Proteinstrukturen an der Virusoberfläche oder der Wirtsoberfläche zurückzuführen, was die Virenadsorption oder -penetration an/ in die Wirtszelle und somit die Virusvermehrung beeinträchtigt. Dabei kann die Virusoberfläche zum einen eine reine Proteinhülle sein. Komplexe Viren sind zum anderen von einer lipidhaltigen Hülle umgeben, die mit Glycoproteinen (Spikes) durchsetzt ist. Diese Glycoproteine sind für die infektiöse Aktivität des Virus mitverantwortlich. Sie werden durch Glucosidasen synthetisiert. Gerbstoffe wirken zum einen durch die Reaktion mit Membranproteinen antiviral, zum anderen wird ihre Toxizität auf die Inhibierung der Glucosidasen und daraus resultierend auf eine Veränderung der zur Infektion nötigen intakten Oberflächenstruktur des Virus zurückgeführt. Dies würde auch erklären, warum umhüllte Viren wie Herpes Simplex empfindlicher gegenüber Gerbstoffen reagieren als hüllenlose Viren.

Die Erfindung wird anhand der Figuren 1 bis 7 näher erläutert. Im Einzelnen zeigen:

Figur 1: Extraktionsschema für die Aufarbeitung getrockneter, gemahlener Rhabarberwurzeln.

Figur 2: Die chromatografische Auftrennung der sc gewonnenen Ethylacetat-Fraktion 4 aus Rheum palmatum MAXIM (Fraktion 4 -Variante11). Die obere Spur zeigt jeweils die Absorption bei 280 nm, die untere bei 242 nm.

Figur 3: Die chromatografische Auftrennung der sc gewonnenen Ethylacetat-Fraktion 5 aus Rheum-spec. Landsorte Polen 1976 (Fraktion 5- Variante 25), Absorption bei 280 nm.

Figur 4: Die chromatografische Auftrennung der sc gewonnenen Ethylacetat-Fraktion 9 aus Rheum-spec. Landsorte Polen 1976 (Fraktion 9- Variante 25), Absorption bei 280 nm.

Figur 5: Inhibierung der Virusaktivität von SFV, PPV, BVDV und PRV durch Rheum Fraktion 4-Variante 11.

Figur 6: Inhibierung der Virusaktivität von SFV, PPV, BVDV und PRV durch Rheum Fraktion 5-Variante 25.

Figur 7: Inhibierung der Virusaktivität von SFV, PPV, BVDV und PRV durch Rheum Fraktion 9-Variante 25.

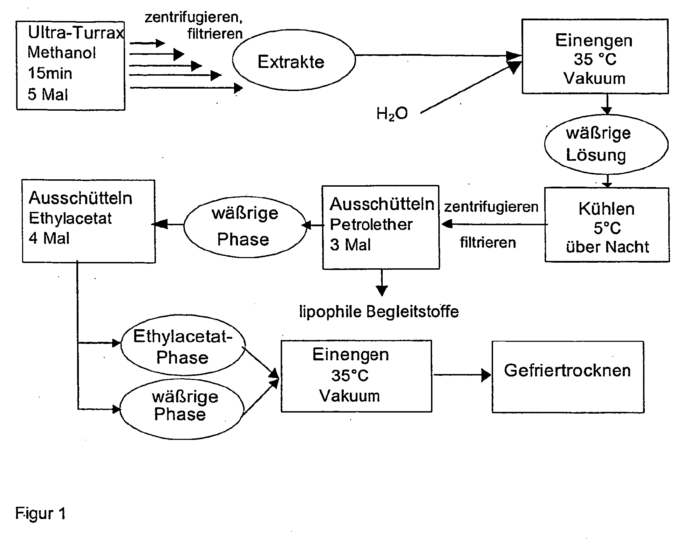

Das Extraktionsschema für die Aufarbeitung getrockneter, gemahlener Rhabarberwurzeln ist in der Figur 1 dargestellt. Zur Gewinnung der Wirkstoffe wird eine bekannte Extraktionsvorschrift, wie sie z.B. in Abb. 8 der DE 10132936A1 dargestellt wird, modifiziert.

Für die Erzeugung der wässrigen Ausgangsphase wird statt Aceton/Wasser Methanol als Primär-Extraktionsmittel eingesetzt, und diese methanolischen Extrakte werden weiterverwendet. Nach der mehrstufigen extraktiven Aufarbeitung wird eine präparative säulenchromatographische Fraktionierung des Ethylacetat-Gesamtextraktes durchgeführt.

Für eine Extraktion des Rhabarberwurzel-Materials wurden 1 kg einer getrockneten und gemahlenen Wurzelprobe einer fünfmaligen Wirbelextraktion mit insgesamt etwa 8000 ml Methanol für je 15 min am Ultra-Turrax unterworfen. Die gesammelten Extrakte wurden dann am Rotationsverdampfer unter Vakuum bei 35°C eingeengt, und nach Zugabe von 500 ml Wasser wurde das Methanol vollständig abgezogen.

Zur Ausfällung störender lipophiler Substanzen wurde der wässrige Extrakt über Nacht unter Stickstoffbegasung im Kühlschrank bei 5 °C aufbewahrt. Die Fällungen konnten am nächsten Tag abzentrifugiert werden. Zur Abtrennung weiterer lipophiler Begleitstoffe wurde die wässrige Lösung anschließend noch drei Mal mit je 500 ml Petrolether ausgeschüttelt. Die so gereinigte wässrige Phase wurde daraufhin vier Mal mit je 750 ml Ethylacetat ausgeschüttelt, wonach sich in den vereinigten Ethylacetat-Phasen vermehrt niedrig oligomerisierte Verbindungen befinden sollten und in der verbleibenden wässrigen Phase Verbindungen mit höherem Oligomerisierungsgrad. Sowohl die Ethylacetat- als auch die Wasser-Phase wurden dann am Rotationsverdampfer unter Vakuum bei 35°C eingeengt, in wenig Wasser aufgenommen und anschließend gefriergetrocknet.

Die gefriergetrockneten gerbstoffangereicherten Extrakte wurden bis zur weiteren Verwendung unter Stickstoffatmosphäre bei -21°C aufbewahrt.

Die säulenchromatografische (sc) präparative Auftrennung der Ethylacetat - sowie der Wasser-Phase erwies sich als unumgänglich, um das Gesamtspektrum an polyphenolischen Verbindungen auf eine begrenzte Anzahl an Substanzen einzuschränken.

Zur präparativen Isolierung von Proanthocyanidinen wird schon seit langem die Trennung an Sephadex LH-20 eingesetzt. Als Elutionsmittel haben sich Ethanol oder Alkohol/Wasser-Gemische etabliert. Zur Trennung polymerer Substanzen sind Aceton/Wasser-Gemische (7 : 3; v/v) geeignet.

Die Ethylacetat-Phase wurde in Methanol bzw. Methanol/Wasser gelöst, auf die Säule (Länge 100 cm, Durchmesser 5 cm) aufgegeben und nacheinander mit Ethanol, Methanol und Aceton/Wasser (7 : 3; v/v) eluiert. Hierfür wurde eine MPLC-Anlage mit Probenaufgabepumpe genutzt. Nach DC Überprüfung der erhaltenen Reagenzglasfraktionen wurden für die einzelnen Genotypen die Fraktionen der Ethylacetat - Phase mit gleichem Inhaltsstoffspektrum zusammengefasst.

Nach der sc Trennung der Ethylacetat-Phase resultierten z.B. beim GT 11 (Rheum palmatum M.) 11 Fraktionen, beim GT 25 (Rheum spec. Landsorte Polen) 14 Fraktionen.

Für den erfindungsgemäßen Einsatz wurden die Fraktionen 4-Variante 11 (Rheum palmatum M.), Fraktionen 5 bzw. 9-Variante 25 (Rheum spec. Landsorte Polen) benutzt.

Für die Charakterisierung der durch säulenchromatografische Vortrennung gewonnenen und hinsichtlich ihrer Wirkung als besonders effektiv einzuschätzenden Fraktionen gelten folgende Bedingungen:

|  |  |  |  |
|---|---|---|---|
| Chromatographie |  |  |  |
| Stationäre Phase: | Synergie 4m Polar RP 80, 250 X 2,0 mm |  |  |
| Mobile Phase: | A | 0,1 %ige Ameisensäure |  |
|  | B | Methanol |  |
| Flow: | 0,3 ml/min |  |  |
| Gradientenbedingungen: |  | Time (min) | %B |
|  |  | 0 | 0 |
|  |  | 10 | 10 |
|  |  | 40 | 50 |
|  |  | 41 | 0 |
|  |  | 55 | 0 |
| Detektion: | DAD |  |  |

Über Kalibration mit entsprechenden Standardsubstanzen konnten folgende Substanzen in den sc vorgetrennten Fraktionen aus der Ethylacetatphase - soweit enthalten - quantifiziert werden:

Epigallocatechim

Catechin
Procyanidin B1
Epicatechin
Procyanidin B2
Epigallocatechingallat
Epicatechingallat.

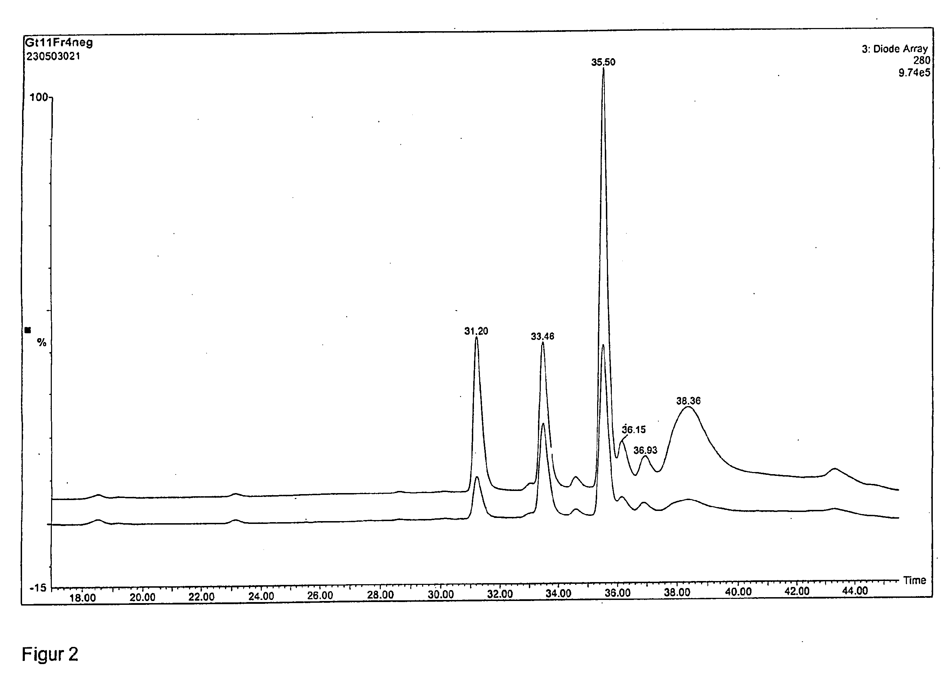

Die Figur 2 zeigt das Chromatogramm der wirksamsten Fraktion aus der sc Trennung der Ethylacetat/Phase von Rheum palmatum MAXIM. Die obere Spur zeigt die Absorption bei 280 nm, die untere bei 242 nm. Es wurden beide Wellenlängen zur Quantifizierung verwendet, da die beiden Procyanidine deutlich höhere Absorptionsintensitäten bei 242 nm aufwiesen.

Bei Rheum palmatum MAXIM. konnten quantitativ bestimmt werden:

Catechin
Procyanidin B1
Procyanidin B2
Epicatechingallat.

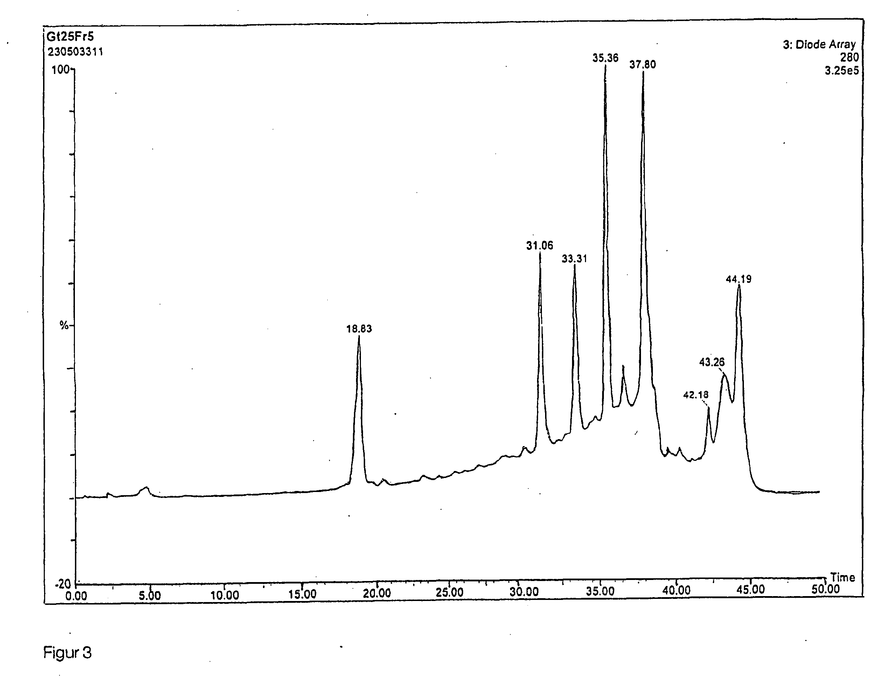

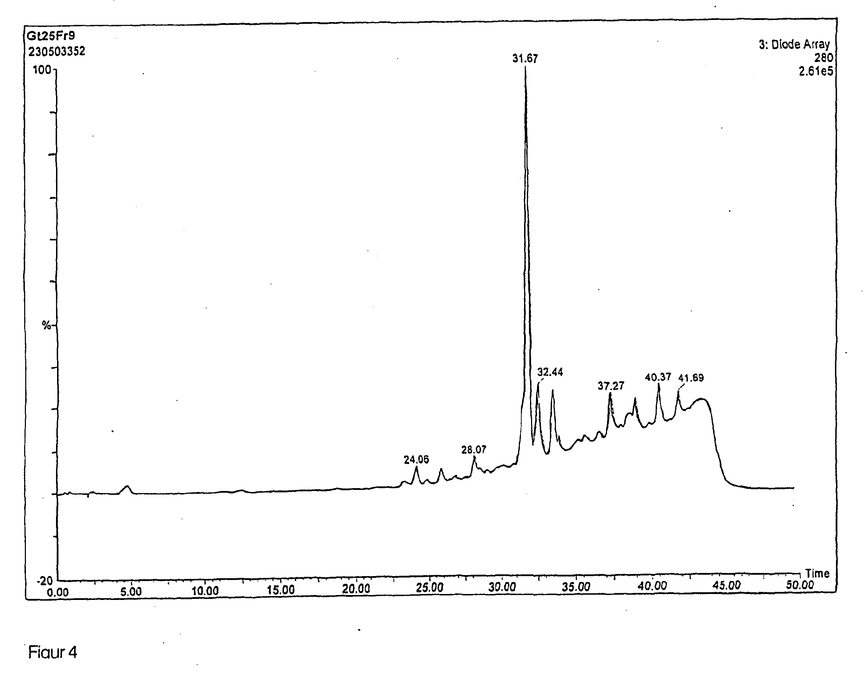

Diese Daten wurden durch gleichzeitige Aufnahme des Totalionenstroms über Massenspektrometrie abgesichert. Die Identifizierung der Substanzen erfolgte außerdem über den Vergleich der Massenspektren der Standardsubstanzen mit den Massenspektren der bei den Standardretentionszeiten detektierten Peaks.
Im Vergleich mit allen anderen Fraktionen wies die Fraktion 4 isoliert aus Rheum palmatum MAXIM sowohl in der Summe die höchsten Gehalte an quantifizierbaren Substanzen auf als auch insbesondere die höchsten Gehalte an Epicatechingallat. Die Abbildungen 3 und 4 zeigen die Chromatogramme der wirksamsten Fraktionen aus der sc Trennung der Ethylacetat/Phase von Rheum spec. Landsorte Polen 1976 (Fraktionen 5 bzw. 9- Variante 25). Diese Art konnte nicht näher spezifiziert werden. Die Chromatogramme stellen die UV-Spur bei 280 nm dar.
Bei Rheum spec. Landsorte Polen 1976 konnten in Fraktion 5 quantitativ bestimmt werden:

Procyanidin B1
Procyanidin B2
Epicatechingallat.

In Fraktion 9 waren die als Standards zu Verfügung stehenden Substanzen nicht mehr enthalten. Über Massenspektrometrie wurden höher oligomere polyphenolische Verbindungen, wie digalloylierte dimere Procyanidine und trimere Procyanidine, mit unterschiedlichem Galloylierungsgrad neben weiteren für Rhabarber beschriebenen Verbindungen, wie z.B. Stilbenderivate, detektiert.
Rheum species ist eine Pflanze, die weltweit vorzufinden ist und sich in verschiedenen Arten seiner Umwelt angepasst hat.
Nachfolgend angeführt ist eine Liste mit allen Rhabarberarten für die erfinderische Lösung.

Variante 01: Rh. officinale BAILL.; Dolomiten (Münster)
Variante 02: Rh. officinale BAILL.; Vacrutot (Ungarn)
Variante 03: Rh. officinale BAILL.; Kirovsk (UdSSR)
Variante 04: Rh. officinale BAILL.; Medicinal Rhubarb, Wisley (England)
Variante 05: Rh. officinale BAILL.; Dolomiten (Münster)
Variante 09: Rh. palmatum L.; Petersburg (UdSSR)
Variante 10: Rh. rhabarbarum L.; The Sutton
Variante 11: Rh. palmatum I.f. florc rubro MAXIM. Petersburg (UdSSR)
Variante 12: Rh. rhabarbarum L.; Holsteiner Blut
Variante 13: Rh. rhabarbarum L.; Utrecht (Holland)
Variante 14: Rh. rhabarbarum L.; Uppsala (Schweden)
Variante 15: Rh. rhabarbarum L.; KVDR 1986
Variante 16: Rh. rhaponticum L.; Kirovsk (UdSSR)
Variante 17: Rh. rhaponticum L.; Dnepropetrovsk (UdSSR)
Variante 18: Rh. rhaponticum L.; Kirovsk (UdSSR)

Variante 19: Rh. rhaponticum L.; Uppsala (Schweden)
Variante 20: Rh. rhaponticum L.; Petrograd (UdSSR)
Variante 21: Rh. rhaponticum L.; Taplozek (Finnland)
Variante 22: Rh. rhabarbarum L.; Kirovsk (UdSSR)
Variante 23: Rh. rhabarbarum L.; China 1956
Variante 24: Rh. rhabarbarum L.; KVDR 1987
Variante 25: Rh. spec. Landsorte Polen 1976
Variante 26: Rh. alexandra VEITCH.; Kirovsk (UdSSR)
Variante 27: Rh. wittrockii LUNDSTR.; Petrograd (UdSSR)
Variante 29: Rh. emod. WOLL.; Petrograd (UdSSR)
Variante 30: Rh. leucorum; Wroclaw (Polen)
Variante 31: Rh. leucorrhizum PALL.; Bukarest (Rumänien)
Variante 32: Rh. maximowiczii LOSINSK.; Petrograd (UdSSR)
Variante 33: Rh. tataricum I.f. Iasi (Rumänien)
Variante 34: Rh. undulatum L.; Pruhonice (CSSR)
Variante 35: Rh. altaicum LOSINSK.; Petrograd (UdSSR)
Variante 36: Rh. altaicum LOSINSK.; Moskau (UdSSR)
Variante 37: Rh. alte Landsorte (Deutschland)
Variante 38: Rh. alte Landsorte (Deutschland)
Variante 39: Rh. alte Landsorte (Polen)
Variante 40: Bastard Rh. rhab. x Rh. raponticum
Variante 41: Rh. alexandra VEITCH.; Petrograd (UdSSR)
Variante 42: Rh. officinale BAILL.; Landsorte, Slovakisches Erzgebirge.

Der Einsatz der erfindungsgemäßen Wirkstofffraktionen wird an folgendem Ausführungsbeispiel näher erklärt:

Für die Versuchsdurchführung wurden folgende Materialen und Methoden zur Virusinaktivierung eingesetzt.

Viren und Zellen

Modellviren für humanpathogene Viren: PPV, BVDV, SFV, PRV
Die in nachfolgender Tabelle aufgeführten Viren wurden für die Inaktivierungsexperimente verwendet. Ebenfalls aufgelistet sind die Zellkulturen, in denen die Viren vermehrt und titriert wurden.

Tabelle 1: Auflistung der für die Experimente eingesetzten Viren, deren Eigenschaften sowie die für die Vermehrung und Titration entsprechenden Zelllinien

| Virus | Familie | Modellvirus für | Genom | Größe [nm] | Zelllinie |
|---|---|---|---|---|---|
| PPV Porcines Parvovirus | Parvoviridae | B19 | ssDNA | 18-26 | PKI3 |
| BVDV Bovine Viral Diarrhea Virus | Flaviviridae | Hepatitis C | ssRNA | 40-50 | MDBK |
| SFV Semliki Forest Virus | Togaviridae | Hepatitis C | ssRNA | 60-80 | Vero |
| PRV Pseudorabies Virus | Herpesviridae | Herpesviren (human) | dsDNA | 150-200 | ML |

Folgende Teilschritte wurden für die Virusinaktivierungsversuche durchgeführt:

Kultivierung der Zellen

Bei den verwendeten Zelllinien (Indikatorzellen, sieheTabelle 2) handelt es sich um adhärente, d.h. in Monolayern wachsende Zellen. Die Zellen unterliegen einer Kontaktinhibition und stellen das Wachstum ein, sobald sie konfluent sind, d.h. ein dichter Zellrasen gewachsen ist. Daher wurden die Zellen subkultiviert, als die Kultur 70 % konfluent war. Die Kultivierung der Zellen erfolgte im Brutschrank bei 37°C, 5 % Kohlendioxid und 95 % relativer Luftfeuchtigkeit. Alle Arbeiten fanden unter sterilen Bedingungen statt. Die Medien wurden vor Gebrauch mit den entsprechenden Zusätzen versehen (siehe Tabelle 2). Alle Zusätze wurden bei-20 °C aufbewahrt. Das fetale Kälberserum (FKS) wurde zusätzlich vor der Verwendung 30 min bei 56°C im Wasserbad Hitze inaktiviert, um vorhandene Komplementfaktoren zu zerstören.

Tabelle 2: Zelllinien zur Vermehrung der Viren

| Zelllinie | Medium | Medienzusätze | Kultivierung in |
|---|---|---|---|
| MDBK Vero ML | DMEM | 0,4% (v/v) NSP 1 % (v/v) L-Glutamin 5% (v/v) FKS | 75 cm$^2$ Zellkulturflaschen |
| PKI3 | DMEM | 0,4 % (v/v) NSP 1 % (v/v) L-Glutamin 10 % (v/v) FKS | 75 cm$^2$ Zellkulturflaschen |

WST-Assay zur Bestimmung der Zytotoxizität

Mit dem Cell-Proliferation Reagent WST-1 kann die metabolische Aktivität lebender, wachstumfähiger Zellen bestimmt werden. Der Test basiert auf der intrazellulären Reduktion von im WST-Reagenz enthaltenem Tetrazoliumsalz in den dunkelroten Farbstoff Formazan durch mitochondriale Dehydrogenasen. Die Farbstoffmenge kann daher direkt mit der Zahl stoffwechselaktiver Zellen korreliert werden.

Eine zytostatische Wirkung lässt sich durch eine verminderte Zellprofiferation nachweisen. Die Absorption wurde mit dem WallacVictor bei einer Wellenlänge von 450 nm bestimmt. In je einer Mikrotiterplatte (96 Kavitäten) wurden pro Kavität 8000 Zellen der Zelllinien PK13, MDBK, Vero und ML ausgesät. Die letzte Spalte (Spalte 12) enthielt nur Medium und diente als Nullprobe. Weiterhin wurden eine Positivkontrolle (nur Medium und Zellen) sowie zwei weitere Kontrollen, in denen das Medium 5 bzw. 10% Ethanol enthielt, mitgeführt. Dies war erforderlich, da die zu untersuchenden polyphenolischen Fraktionen zur besseren Löslichkeit mindestens einen Anteil von 5 % Ethanol im Lösungsmittel benötigten.

Es wurden drei Rhabarber-Fraktionen getestet: Fraktion 4 isoliert aus der Rheum Variante 11, Fraktion 5 und Fraktion 9 isoliert aus der Rheum Variante 25 in den Konzentrationsstufen 0,0025 %, 0,005 %, 0,01 %, 0,05 %, 0,1 % und 0,5 %. Je 50 $\mu$l der jeweiligen Konzentrationsstufe wurden je Fraktion als Doppelbestimmung zu den verschiedenen Zellen pipettiert (ausgenommen der Kontrollen) und diese für 24 Stunden inkubiert. Dann wurden pro Kavität 20 $\mu$l WST-1 -Reagenz hinzu pipettiert und sofort die Absorption gemessen. Anschließend erfolgte die Messung der Absorption alle 30 min für die Zeit von 240 min.

Bestimmung des Virustiter von PPV, BVDV, SFV und PRV

Die Bestimmung des Virustiters erfolgte mittels Endpunkttitration. Hierzu wurde das virushaltige Material so lange verdünnt, bis kein cytopathischer Effekt (CPE) mehr erkennbar war. Die Bestimmung des Virustiter gliedert sich in die nachfolgenden 4 Arbeitsschritte.

1. Aussaat der Zellen

Die Bestimmung des Virustiters erfolgte durch Infektion der entsprechenden Zellen (Tabelle 3) mit verschiedenen Verdünnungen der jeweiligen Virusprobe. Für jede entnommene Probe wurde eine Mikrotiterplatte benötigt. Am Tag vor der Behandlung des jeweiligen Virus mit den Polyphenolen wurden die Zellen in die Mikrotiterplatten eingesät. Für eine Mikrotiterplatte wurden 15 ml Zellsuspension bei einer Einsaat von 150 $\mu$l pro Kavität benötigt. Die Platten wurden im Brutschrank (37 °C, 5 % CO2, 95 % relative Luftfuchtigkeit) aufbewahrt.

2. Herstellung der Verdünnungsreihen für die Titration

Es wurden serielle 1 : 5 Verdünnungen in DMEM (1 % Glutamin, 0,4 % NSP, kein FKS) von den zu untersuchenden Virusproben hergestellt. In einem 96er Probenröhrchen-Rack (8x12 Röhrchen) wurden je 600 $\mu$l DMEM in den Röhrchen (außer Reihe 1) vorgelegt. Reihe 1 enthielt 1 ml der zu untersuchende Virusprobe bzw. 500 $\mu$l Virus plus 500 $\mu$l der jeweiligen Konzentrationsstufe der polyphenolischen Fraktionen. Von Reihe 1 ausgehend, wurden mit einer

Mehrkanalpipette je 150 $\mu$l in die zweite Reihe pipettiert, dreimal gut durchmischt und daraus wiederum 150 $\mu$l in die dritte Reihe pipettiert usw. Bei jedem Verdünnungsschritt erfolgte ein Wechsel der Spitzen. Die 12. Reihe enthielt nur Medium und diente als Negativkontrolle.

3. Titration

Die Titration erfolgte in den am Vortag eingesäten Mikrotiterplatten. Es wurden je 50 $\mu$l der einzelnen Verdünnungsstufen aus dem 96er Probenröhrchen-Rack auf die Mikrotiterplatten aufgetragen. Für jede Verdünnung wurden 8 Replikate getestet. Die Platten wurden 2-5 Tage inkubiert, abhängig von der Inkubationszeit des behandelten Virus. Anschließend erfolgte die mikroskopische Untersuchung auf cytopathische Effekte (CPE).

4. Bestimmung der Tissue Culture Infective Dose; TCID50/ml

Die Platten wurden nach der Inkubationszeit mikroskopisch auf cytopathische Effekte (CPE), die durch Virusinfektionen hervorgerufen wurden, untersucht. Positive Reaktionen in den einzelnen Kavitäten der Mikrotiterplatten wurden im Protokoll vermerkt.
Die Berechnung wurde computergestützt auf der Basis der Probitanalyse durchgeführt. Vereinfacht kann für die Bestimmung des Virustiters die Formel nach Spearman und Kärber angewendet werden:

$$\log_{10} TCID_{50}/\text{Volumen} = X_0 - d/2 + d/n * \sum X_i$$

$X_0 =$     Logarithmus der kleinsten Verdünnung, bei der alle Testobjekte positiv reagieren
$d =$     der Dosis (verdünnungs) abstand in $\log_{10}$
$n =$     die Zahl der pro Verdünnung eingesetzten Testobjekte
$X_i =$     die Summe aller auf die Infektion positiv reagierender Testobjekte ab und einschließlich $X_0$

Der Virustiter wurde für das Volumen 1 Milliliter berechnet. Da zur Endpunktbestimmung ein Volumen von 50 $\mu$l (= 1/20 ml) eingesetzt wurde, musste das Ergebnis noch mit $\log_{10}$ 20 addiert werden. Ebenfalls war bei der Berechnung zu berücksichtigen, dass die Viren durch die Zugabe der Polyphenole 1:1 verdünnt waren.

Ergebnisse der Virusinaktivierung

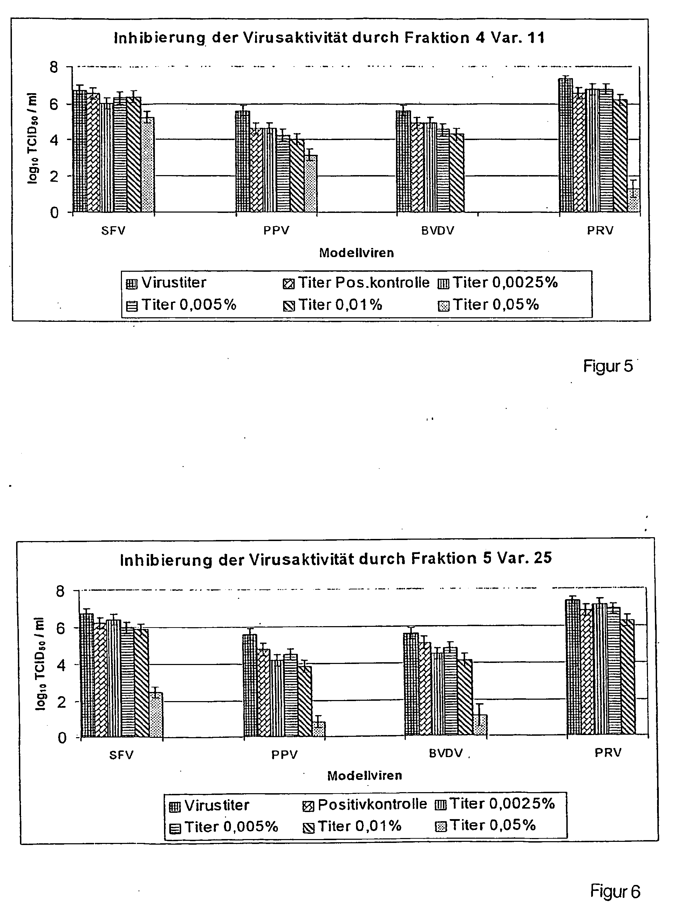

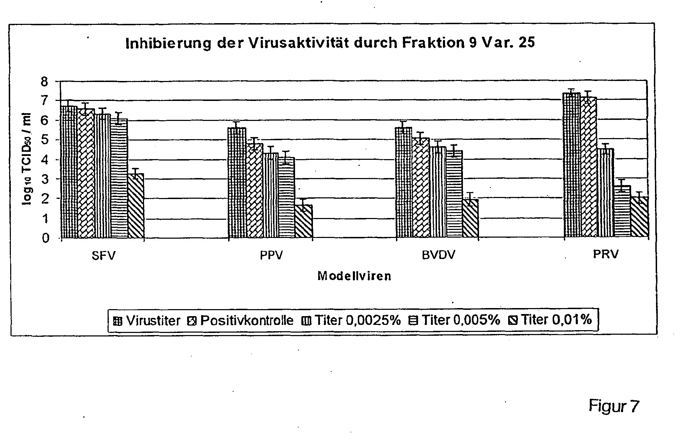

Die in den Ausgangssuspensionen vorhandenen Mengen infektiöser viraler Partikel wurden wie unter 3. beschrieben im klassischen Titrationsverfahren gemessen. Dabei wird jeweils die höchste Verdünnung der Virussuspensiön ermittelt, die in der Kultur der Indikatorzellen zu 50 % einen zytopathologischen Effekt (CPE) verursacht (50% tissue culture infective doses pro ml (log $TCID_{50}$/ml)). In den nachfolgenden Abbildungen sind die log $TCID_{50}$/ml -Werte der Virensuspensionen, der Kontrollen (Positivkontrolle: Virus in Medium mit 5 % Ethanol) sowie der mit den unterschiedlichen Konzentrationsstufen der Rheum-Fraktionen behandelten Viren dargestellt.
Es werden jeweils nur die Konzentrationsstufen gezeigt, bei denen keine Zytotoxizität gegenüber den Indikatorzellen VERO, PK 13, MDBK und ML beobachtet wurde (diese Ergebnisse sind hier nicht aufgeführt).
Wie in Figur 5 dargestellt, werden alle Modellviren bei einer Konzentration von 0,05 % durch die Fraktion 4-Variante11 signifikant gehemmt. Dabei scheinen PRV und PPV die empfindlicheren Viren zu sein, da bereits bei 0,01 % eine signifikante Hemmung gegenüber der reinen Virussuspension zu beobachten ist. Fraktion 5-Variante 25 wirkt am stärksten inhibierend auf PPV und BVDV Alle dargestellten Konzentrationen von 0,05 bis 0,0025 % (Figur 6) zeigen eine Virus inhibierende Wirkung im Vergleich zum Virustiter. Auf PRV wirkt nur die Konzentration 0,01 % Virus inhibierend.
Die antivirale Wirksamkeit der Fraktion 5-Variante 25 ist bei dem Virusmodell SFV im Konzentrationsbereich 0,05 bis 0,005 % zu beobachten.
Die Fraktion 9-Variante 25 wirkt generell schon ab einer Konzentration von 0,05 % zytotoxisch. Die stärksten antiviralen Effekte für diese Fraktion sind bei PRV zu sehen. Wie in Figur 7 dargestellt, tritt bereits bei der niedrigsten eingesetzten Konzentration eine Reduktion der viralen Aktivität um ca. 40 % auf. Bei der höchsten nicht zytotoxischen Konzentration wird eine Reduktion auf ca. 70 % der Ausgangsaktivität erreicht.
Bei PPV und BVDV tritt bei den Konzentrationen 0,01 % bis 0,0025 % eine
Hemmung der viralen Aktivität auf, die bei den höchsten eingesetzten Konzentrationen um ca. 65 bis 70 % gesenkt wird. Für SFV wirkt nur die Konzentration 0,01 % signifikant Virustiter reduzierend.

## EP 2 001 494 B1

**Patentansprüche**

1. Wirkstofffraktionen mit hoher antiviraler Aktivität zur Virusinaktivierung und Desinfektion aus Rheum-species-Arten, erhältlich durch ein Verfahren, umfassend die folgenden Schritte:

   a) Extraktion der getrockneten und gemahlenen Wurzelproben mit Methanol,
   b) Befreiung der gebildeten wässrigen Phase von lipophilen Begleitstoffen unter vollständiger Methanolentfernung nach Zugabe von Wasser,
   c) Ausschütteln der gereinigten wässrigen Phase mit Ethylacetat,
   d) säulenchromatografische Auftrennung der vereinigten Ethylacetatphase in eine begrenzte Anzahl von Wirkstofffraktionen,
   e) Zusammenfassung der Wirkstofffraktionen mit gleichem Inhaltsstoffspektrum,
   f) Abtrennung einzelner Wirkstofffraktionen zur Verwendung nach Durchführung eines Wirksamkeitsnachweises der Wirkstofffraktionen.

2. Wirkstofffraktionen nach Anspruch 1,
   **dadurch gekennzeichnet,**
   **dass** in Schritt b) die Befreiung der gebildeten wässrigen Phase von lipophilen Begleitstoffen durch Ausfällung der lipophilen Begleitstoffe bei 5 °C unter Stickstoffbegasung erfolgt.

3. Wirkstofffraktionen nach Anspruch 1 oder 2,
   **dadurch gekennzeichnet,**
   **dass** in Schritt b) die wässrige Phase mit Petrolether ausgeschüttelt wird.

4. Wirkstofffraktionen nach einem der Ansprüche 1 bis 3,
   **dadurch gekennzeichnet,**
   **dass** nach dem Ausschütteln der gereinigten wässrigen Phase mit Ethylacetat die vereinigten Ethylacetatphasen unter Vakuum eingeengt, in wenig Wasser aufgenommen und anschließend gefriergetrocknet werden.

5. Wirkstofffraktionen nach einem der Ansprüche 1 bis 4,
   **dadurch gekennzeichnet,**
   **dass** in Schritt d) zur säulenchromatogräfischen Auftrennung als Elutionsmittel Ethanol, Methanol und ein Gemisch aus Aceton und Wasser eingesetzt werden.

**Claims**

1. Active substance fractions from *Rheum-species* species, having high anti-viral activity for the inactivation of viruses and disinfection and obtainable by a method comprising the following steps:

   a) extracting dried and ground root samples with methanol,
   b) removing accompanying lipophilic substances from the aqueous phase produced, with complete removal of methanol, after the addition of water,
   c) extracting the purified aqueous phase with ethyl acetate,
   d) fractionating the combined ethyl acetate phase by column chromatography to give a limited number of active substance fractions,
   e) combining the active substance fractions having the same spectrum of constituents,
   f) removing individual active substance fractions for use after assaying the efficacy of the active substance fractions.

2. Active substance fractions according to Claim 1,
   **characterized in that**
   the accompanying lipophilic substances are removed in step b) from the aqueous phase produced by precipitating said accompanying lipophilic substances at 5°C while gassing with nitrogen.

3. Active substance fractions according to Claim 1 or 2, **characterized in that**
   the aqueous phase is extracted with petroleum ether in step b).

**4.** Active substance fractions according to any of Claims 1 to 3, **characterized in that**
after extracting the purified aqueous phase with ethyl acetate, the combined ethyl acetate phases are concentrated *in vacuo*, taken up in a little water and then freeze-dried.

**5.** Active substance fractions according to any of Claims 1 to 4, **characterized in that**
the eluants used in step d) for fractionation by column chromatography are ethanol, methanol and a mixture of acetone and water.

**Revendications**

**1.** Fractions de principe actif à puissante activité antivirale destinées à l'inactivation virale et à la désinfection, issues de l'espèce *Rheum*, pouvant être obtenues par un procédé comportant les étapes suivantes :

a) extraction des échantillons de racine séchée et broyée avec du méthanol,
b) élimination depuis la phase aqueuse formée, des impuretés d'accompagnement lipophiles avec élimination totale du méthanol après ajout d'eau,
c) extraction de la phase aqueuse purifiée à l'acétate d'éthyle,
d) séparation par chromatographie en colonne des phases acétate d'éthyle réunies en un nombre délimité de fractions de principe actif,
e) regroupement des fractions de principe actif présentant un même spectre d'ingrédients,
f) séparation des fractions de principe actif individuelles pour utilisation après réalisation d'une détection d'efficacité des fractions de principe actif.

**2.** Fractions de principe actif selon la revendication 1, **caractérisées en ce que** dans l'étape b), la libération depuis la phase aqueuse formée des impuretés d'accompagnement lipophiles se fait par précipitation des impuretés d'accompagnement lipophiles à 5 °C sous gazage à l'azote.

**3.** Fractions de principe actif selon la revendication 1 ou 2, **caractérisées en ce que** dans l'étape b), la phase aqueuse est extraite à l'éther de pétrole.

**4.** Fractions de principe actif selon l'une des revendications 1 à 3, **caractérisées en ce qu'**après l'extraction de la phase aqueuse purifiée à l'acétate d'éthyle, les phases réunies d'acétate d'éthyle sont concentrées sous vide, reprises dans un peu d'eau et lyophilisées.

**5.** Fractions de principe actif selon l'une des revendications 1 à 4, **caractérisées en ce que** dans l'étape d), pour la séparation par chromatographie en colonne, on utilise comme éluant de l'éthanol, du méthanol et un mélange d'acétone et d'eau.

Figur 1

Figur 2

Figur 3

EP 2 001 494 B1

Figur 4

Figur 5

Figur 6

**Inhibierung der Virusaktivität durch Fraktion 9 Var. 25**

Legend: ⊞ Virustiter  ⊠ Positivkontrolle  ⊞ Titer 0,0025%  ⊟ Titer 0,005%  ⊠ Titer 0,01%

Y-axis: $\log_{10}$ TCID$_{50}$ / ml

X-axis: Modellviren (SFV, PPV, BVDV, PRV)

Figur 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10132936 A1

**In der Beschreibung aufgeführte Nicht-Patentliteratur**

- **Esquenazi, D. ; Wigg, M. D.** *Research in Microbiology,* 2002, vol. 153, 647-652
- **Cheng, H. Y. ; Lin, C. C.** *Antivir Chem Chemother.,* 2002, vol. 13 (4), 223-229
- **Cheng, H. Y. ; Un, T.C.** *Planta Med.,* 2003, vol. 69 (10), 953-956
- **Weber.** *Antiviral Res.,* 2003, vol. 58 (2), 167-173
- **Shahat, A. A.** *Planta Med,* 2002, vol. 68, 539-541
- **L. wurde von Sokmen.** *Ufe Sciences,* 2005, vol. 76, 2981-2993
- **Wang, Z. W.** *Zhongguo Zhong Yao Za Zhi,* 1996, vol. 21 (6), 364-366